# FASCICULE DE BREVET EUROPEEN

(11) **EP 1 475 045 B1**
(45) Date de publication et mention de la délivrance du brevet: **07.05.2008**
(21) Numéro de dépôt: 04356040.8
(22) Date de dépôt: 26.03.2004
(51) Int. Cl.: A61B 17/04, A61F 2/00

(54) **Introducteur et guide perforateur pour la mise en place d'une bandelette dans le corps humain**
Einführungsvorrichtung und Bohrführung zum Anbringen von einem Band in den menschlischen Körper
Introducer and drill guide for delivering a strip into the human body

(30) Priorité: 28.03.2003 FR 0303897
(43) Date de publication de la demande: 10.11.2004
(73) Titulaire: Analytic Biosurgical Solutions - ABISS, 42000 St Etienne (FR)
(72) Inventeur: Beraud, Jean-Marc, 42100 St Etienne (FR)
(74) Mandataire: Blanchard, Eugène Gilles

(56) Documents cités:
- WO-A-00/74633
- FR-A- 2 804 010
- US-A- 5 112 344
- US-B1- 6 319 272

## Description

La présente invention concerne le domaine technique de l'insertion de bandelettes de renfort pour venir conforter des tissus altérés du corps humain.

Dans une application préférée, l'invention concerne le domaine des bandelettes de renfort utilisées dans la chirurgie uro-gynécologique pour le traitement de l'incontinence d'efforts chez la femme.

Il est connu de traiter l'incontinence urinaire d'efforts de la femme au moyen d'une bandelette de support qui est placée sous l'urètre, de manière à en assurer le soutien.

A cet effet, une demande de brevet FR 2 804 010 a, par exemple, proposé de mettre en oeuvre, en tant qu'élément de support, une bandelette en matériau biocompatible tissé ou tricoté, tel que, par exemple, du polypropylène monofil tricoté. Cette bandelette est alors placée sous l'urètre de la patiente à traiter, au moyen d'un introducteur qui présente un corps souple allongé, pourvu, à chacune de ses deux extrémités, de deux moyens de traction qui définissent, entre ses extrémités, une cavité de réception étanche à l'intérieur de laquelle une bandelette de renfort est disposée. Selon ce document, la cavité et le corps de l'introducteur sont réalisés par l'assemblage de deux demi-corps au moyen d'un élément de liaison présentant, en tant que moyen de découpe, une zone de plus faible résistance, susceptible de se rompre sous l'effet d'une traction conjointe sur les moyens de traction d'extrémités du corps.

Un tel introducteur permet, effectivement, d'assurer la mise en place de la bandelette de renfort mais présente, toutefois, l'inconvénient d'être particulièrement difficile à découper, dans la mesure où il est nécessaire d'effectuer une traction importante afin de déchirer le corps de l'introducteur au niveau de ses moyens de découpe et, dans le cas où il n'a pas été possible d'assurer cette découpe spontanée par simple traction, il est nécessaire de recourir à un outil de coupe au risque d'endommager la bandelette de renfort.

De plus, la nécessité d'effectuer une traction importante sur l'introducteur présente le risque de précontraindre la bandelette de renfort, de sorte que cette dernière relève trop l'urètre de la patiente empêchant alors, soit une vidange complète de la vessie, soit toute vidange de la vessie imposant alors une intervention chirurgicale corrective supplémentaire.

Ainsi, il apparaît le besoin d'un nouveau dispositif pour la mise en place d'une bandelette de renfort qui offre des moyens de découpe faciles à mettre en oeuvre et qui garantissent une dépose de la bandelette in situ sans précontrainte.

Afin d'atteindre cet objectif, l'invention concerne un dispositif pour la mise en place d'une bandelette de renfort dans un tissu du corps humain comprenant un introducteur qui présente :
■ un corps souple allongé,
■ à chacune des deux extrémités du corps, des moyens de traction,
■ entre les deux extrémités, une cavité de réception de la bandelette,
■ et, au niveau de la cavité, des moyens de découpe du corps en deux parties séparables par une traction exercée sur les moyens de traction.

Selon l'invention, le dispositif de mise en place est caractérisé en ce que les moyens de découpe comprennent au moins une ouverture aménagée dans la paroi de la cavité et destinée au passage d'un outil de coupe.

La mise en oeuvre d'une telle ouverture permet de réaliser la découpe du corps de l'introducteur, au niveau de la cavité de réception de la bandelette, donc sans risque d'entamer cette dernière ou de l'abîmer, dans la mesure où cette découpe est procédée par l'ouverture, offrant au chirurgien un contrôle visuel de la position de la bandelette à l'intérieur de la cavité.

Selon l'invention, les moyens de découpe comprennent au moins une ou plusieurs ouvertures et, selon une forme de réalisation, les moyens de découpe comprennent au moins deux ouvertures disposées en regard l'une de l'autre.

Selon une autre caractéristique de l'invention, la ou les ouvertures sont disposées de manière à permettre la mise en place de la bandelette dans la cavité de l'introduction.

Cette caractéristique, particulièrement avantageuse, permet au chirurgien de choisir le type de bandelettes de renfort qu'il utilisera au moment même de l'intervention.

Ainsi, le chirurgien peut alors choisir de mettre en oeuvre, soit une bandelette en matériau synthétique biocompatible soit, au contraire, une bandelette en matériau naturel biocompatible, tel que, par exemple, du « fascia latta » ou, encore, un produit vendu sous la marque PELVICOL.

Selon une autre caractéristique de l'invention, la paroi de la cavité présente une série de perforations qui permettent de recourir à une stérilisation de l'introducteur et de son contenu éventuel à la vapeur.

Selon une autre caractéristique de l'invention, le dispositif de mise en place comprend une bandelette, disposée à l'intérieur de la cavité de l'introducteur, en étant libre, c'est-à-dire en ne présentant aucune liaison mécanique positive avec l'introducteur et, plus particulièrement sa cavité de réception, de sorte que les contraintes, appliquées à l'introducteur, ne sont pas répercutées à la bandelette.

Selon l'invention, les moyens de traction de l'introducteur peuvent être réalisés de toute façon appropriée et peuvent être amovibles ou non.

Selon une forme préférée de réalisation, les moyens de traction comprennent des aiguilles semi-rigides ou semi-souples, solidaires des extrémités du corps allongé de l'introducteur.

L'introducteur selon l'invention peut être utilisé pour la mise en place d'une bandelette de renfort selon les différentes voies et procédures connues telles que décrites par exemple mais non exclusivement dans la demande de brevet FR 2 804 010 ou encore la demande de brevet US 2002099260.

Afin de faciliter le travail du chirurgien, selon une autre caractéristique de l'invention, le dispositif de mise en place comprend également au moins un ancillaire qui comprend un guide perforateur allongé ou trocart dont une extrémité est destinée à être introduite dans le corps de la patiente et dont l'autre extrémité est pourvue d'une poignée.

Selon une caractéristique de l'invention, le guide perforateur présente une forme arquée dans un plan. De manière préférée mais non strictement nécessaire, la partie arquée du perforateur s'étend sur un secteur angulaire supérieur à 140° et, de préférence mais non nécessairement, inférieur à 180° et, de préférence, compris entre 150° et 170°. De manière préférée, la partie arquée du guide perforateur présente alors un rayon de courbure compris entre 30 mm et 60 mm et, de préférence, pour la partie du guide perforateur s'étendant entre la poignée et l'extrémité destinée à être introduite dans le corps du patient, compris entre 40 mm et 50 mm, la partie extrême du perforateur présentant alors un rayon de courbure variable.

Selon une autre forme de réalisation, le guide perforateur présente, au niveau de son extrémité opposée à la poignée ou distale, une forme hélicoïdale. De manière préférée, le guide perforateur présente alors la forme d'une portion de spire hélicoïdale s'étendant sur un angle compris entre 180° et 360° et, de préférence, compris entre 255° et 270°. De même, de manière préférée, la spire du guide perforateur présente un rayon de courbure compris entre 20 mm et 40 mm, avec un pas compris entre 15 mm et 25 mm.

Selon une autre caractéristique de l'invention, afin de réduire les traumatismes subis par le corps de la patiente lors de l'introduction de l'implant, le dispositif d'introduction comprend en outre une chemise tubulaire de forme complémentaire à celle du guide perforateur. Cette chemise tubulaire est alors destinée à être engagée sur le guide perforateur et à rester dans le corps de la patiente après retrait du guide perforateur pour définir un tunnel pour le passage des moyens de traction de l'introducteur. La chemise tubulaire est ensuite retirée, après passage des moyens de traction lors du retrait de l'introducteur.

Selon l'invention, la chemise tubulaire peut être réalisée en tout matériau souple biocompatible, tel que, par exemple mais non exclusivement, du PVC.

Selon l'invention, la chemise tubulaire peut présenter soit une longueur sensiblement égaie à celle de la partie utile du guide perforateur soit une longueur supérieure à celle de la longueur utile du guide perforateur. Dans ce dernier cas la chemise tubulaire présentera une ouverture latérale aménagée à une distance d'une extrémité libre de la chemise inférieure ou égale à la longueur de la partie utile du guide perforateur de manière à permettre une mise en place du guide perforateur dans la chemise en laissant dépasser la pointe dudit perforateur hors de la chemise.

Diverses autres caractéristiques de l'invention ressortent de la description ci-dessous effectuée en référence aux dessins annexés qui illustrent différentes formes de réalisation d'un introducteur selon l'invention, ainsi que de guides perforateurs permettant de faciliter la mise en oeuvre de l'introducteur conforme à l'invention.

Par ailleurs, il doit être noté que les différentes caractéristiques de l'invention, décrites précédemment et ci-après, peuvent être combinées ensemble selon différentes variantes, en fonction de la pathologie à traiter.

La **fig. 1** est une vue de dessus d'un introducteur pour la mise en place d'une bandelette de renfort selon l'invention.

La **fig. 2** est une coupe partielle selon la ligne **II-II** de la **fig. 1****.**

La **fig. 3** est une coupe, analogue à la **fig. 2**, montrant l'introducteur conforme à l'invention en position pliée, de manière à permettre l'introduction d'une bandelette dans la cavité de réception de l'introducteur.

La **fig. 4** est une vue de côté d'une autre forme de réalisation d'un introducteur selon l'invention.

La **fig. 5** est une coupe partielle selon la ligne **V-V** de la **fig. 4**.

La **fig. 6** est une élévation, partiellement arrachée, d'un guide perforateur pouvant être utilisé pour la mise en place de l'implant selon l'invention et présentant une forme arquée.

La **fig. 7** est une élévation d'une autre forme de réalisation d'un guide perforateur selon l'invention, présentant une extrémité d'introduction de forme hélicoïdale.

La **fig. 8** est une vue de gauche du guide perforateur selon la **fig. 3****.**

La **fig. 9** est une vue de dessous du perforateur illustré à la **fig. 3****.**

Les **fig. 10** à **13** sont des vues, analogues aux **fig. 6** à **7**, montrant des variantes de réalisation de guides perforateurs pour la mise en place d'un implant conforme à l'invention.

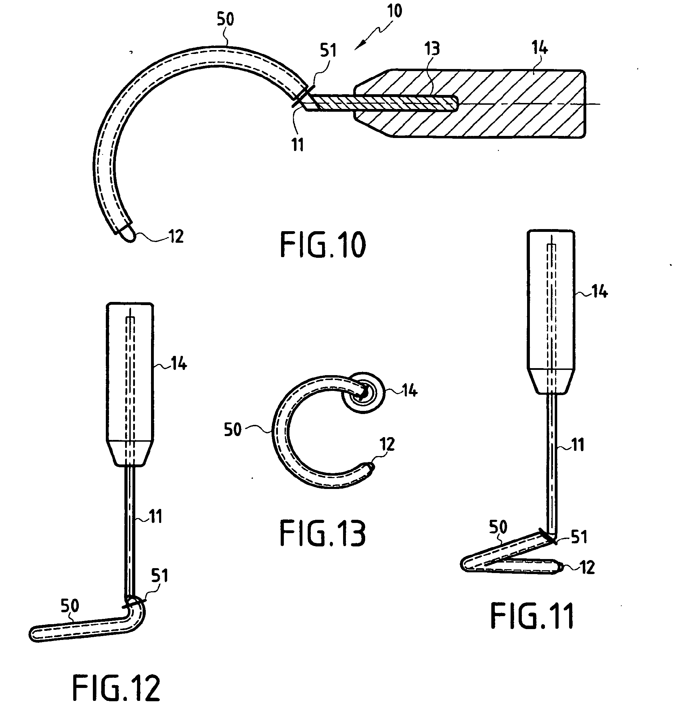

La **fig .14** est une vue analogue à la **fig.10** montrant encore une autre variante de réalisation d'un guide perforateur selon l'invention.

L'invention vise à offrir des moyens permettant de faciliter le travail d'un chirurgien pour la mise en place d'une bandelette de support, utilisée pour le traitement, par exemple mais non exclusivement, de l'incontinence d'effort.

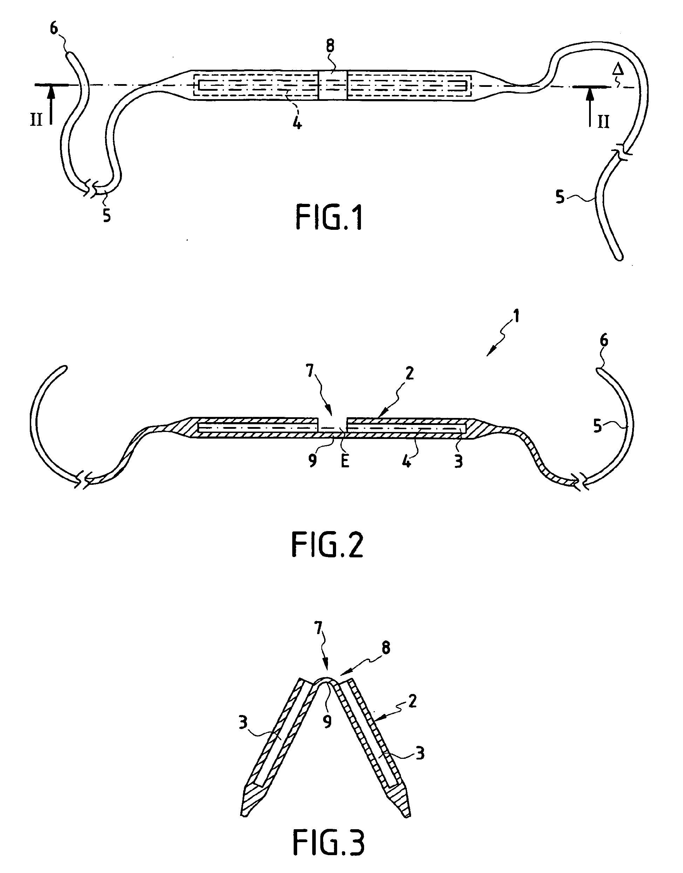

A cet effet, l'invention propose un dispositif pour la mise en place d'une telle bandelette qui comprend, tout d'abord, un introducteur, tel qu'illustré aux **fig. 1** et **2** et désigné dans son ensemble par la référence **1**.

Un tel introducteur **1** comprend un corps souple allongé **2** qui définit une cavité **3** de réception d'une bandelette de renfort **4**, matérialisée de manière schématique en traits mixtes. L'introducteur **1** comprend, au niveau de chacune des deux extrémités du corps **2**, des moyens de traction **5** qui peuvent être réalisés de toute façon appropriée, en étant amovibles ou non.

Selon l'exemple illustré, les moyens de traction **5** sont constitués, pour chaque extrémité du corps souple, par une aiguille semi-rigide ou semi-souple, présentant une extrémité d'introduction **6** réalisée sous la forme d'une pointe mousse, c'est-à-dire atraumatique, n'étant pas susceptible de couper ou blesser les tissus dans lesquels elle doit être introduite.

Le corps souple **2** et les moyens de traction **5** peuvent être réalisés en tout matériau compatible et, de préférence dans un matériau polymère synthétique de la famille des plastiques à faible coefficient de friction, tels que, par exemple, le polyéthylène. L'aiguille **5** sera alors, de préférence, réalisée dans le même matériau que le corps **2**, sans toutefois que cette caractéristique puisse être considérée comme strictement nécessaire à la réalisation d'un introducteur **1** conforme à l'invention.

Selon une caractéristique essentielle de l'invention, l'introducteur **1** comprend, enfin, des moyens de découpe **7** qui comprennent au moins une, et, selon l'exemple illustré, exactement une ouverture **8**, aménagée dans le corps souple **2** au niveau de la cavité **3**. Selon l'exemple illustré, l'ouverture **8**, constitutive des moyens de découpe **7**, s'étend transversalement à l'axe longitudinal Δ de la cavité **3** et intéresse plus de la moitié de la circonférence de la paroi de la cavité, de manière à ne laisser subsister qu'une paroi **9** de liaison entre les deux parties du corps **2** délimitées par l'ouverture **8**.

Cette caractéristique de l'invention permet au chirurgien de découper la paroi du demi-corps **2** en venant placer la pointe d'un outil de coupe, tel qu'une paire de ciseaux, dans l'espace **E**, entre la bandelette **4** et la paroi **9**, en pouvant contrôler la position exacte de la bandelette **4** et donc ne pas risquer de couper accidentellement cette dernière.

De plus, cette forme particulière de réalisation des moyens de découpe permet de plier la bandelette au niveau de la paroi **9**, comme illustré à la **fig. 3****,** de manière à pouvoir placer dans la cavité **3** n'importe quel type de bandelette, en fonction de la pathologie à traiter. Ainsi, l'introducteur **1** selon l'invention n'est pas nécessairement fourni au chirurgien avec la bandelette de renfort **4** disposée à l'intérieur de la cavité **3**.

Le dispositif introducteur conforme à l'invention peut ainsi être utilisé pour tout type de bandelettes en matériau synthétique ou en matériau naturel.

Toutefois, selon une caractéristique de l'invention, l'introducteur **1** comprend une bandelette prédisposée dans la cavité 3 et en étant libre par rapport aux parois de cette dernière, de sorte que les contraintes de traction, appliquées à l'introducteur **1** ne sont pas répercutées à la bandelette qu'il contient, permettant ainsi de déposer cette dernière dans un état détendu sans précontrainte.

Selon l'exemple illustré aux **fig. 1** à **3**, les moyens de découpe **7** sont constitués par une unique ouverture **8** aménagée dans la paroi du corps **2** au niveau de la cavité **3**. Cependant, un tel mode de réalisation des moyens de découpe **7** n'est pas strictement nécessaire à la réalisation d'un introducteur **1** conforme à l'invention.

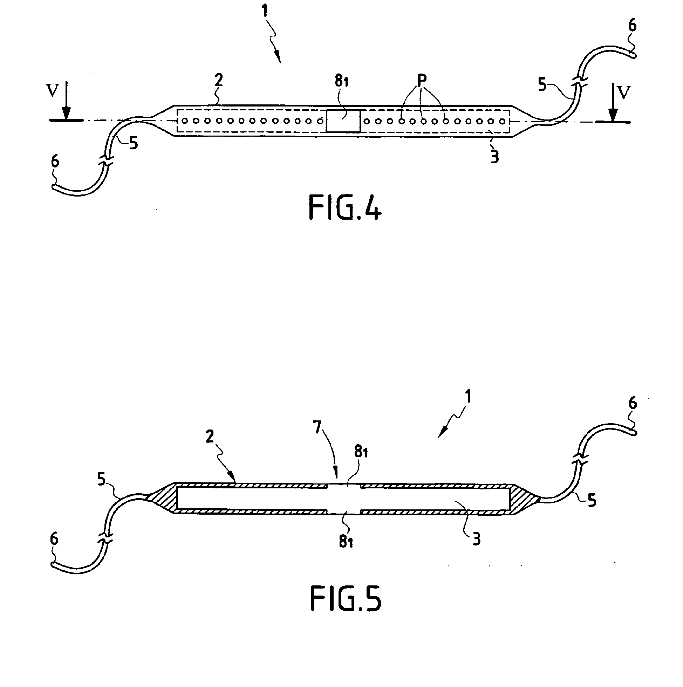

Ainsi, les **fig. 4** et **5** illustrent une autre forme de réalisation d'un introducteur **1** selon l'invention pour lequel les moyens de découpe **7** sont constitués par deux ouvertures **8**, **8₁,** aménagées dans le corps **2** au niveau de la cavité **3,** de manière à être en regard l'une de l'autre. Par ailleurs, selon cet exemple de réalisation, l'introducteur **1** présente des micro-perforations **P** réalisées dans la paroi de cavité **3** pour permettre une stérilisation de l'intérieur de cette dernière et de son éventuel contenu.

La mise en oeuvre d'un introducteur **1** selon l'invention présente, de plus, l'avantage de permettre de réduire au maximum l'abrasion des tissus musculaires traversés lors de la mise en place de la bandelette **4**.

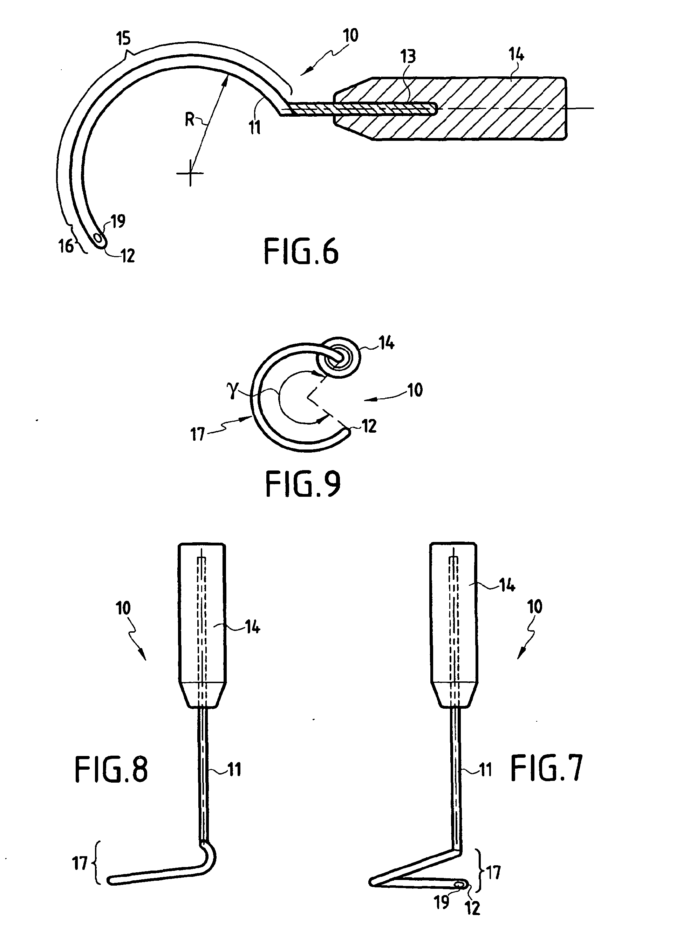

Dans le même sens, afin de réduire au minimum la dissection de la région de mise en place de la bandelette et donc le traumatisme en découlant, l'invention propose au chirurgien procédant au traitement d'utiliser un ou plusieurs guides perforateurs allongés **10**, tels que ceux plus particulièrement illustrés aux **fig. 6** et à **9**.

De manière générale, un tel guide perforateur **10** comprend un corps ou mandrin allongé **11** dont une extrémité **12** est destinée à être introduite dans le corps du sujet à traiter et dont l'autre extrémité **13** est pourvue d'une poignée **14**. Il doit être remarqué que l'extrémité d'introduction **12** est, de préférence, constituée par une pointe mousse, c'est-à-dire une pointe atraumatique qui n'est pas susceptible de blesser ou de couper les tissus dans lesquels elle doit être introduite.

Selon une forme de réalisation illustrée à la **fig. 6****,** le guide perforateur **10** présente une forme arquée dans un plan. Cette forme arquée dans un plan est plus particulièrement adaptée pour la mise en place des bretelles de suspension dans les zones supérieure et inférieure des trous obturés. De manière préférée mais non strictement nécessaire la partie arquée du guide perforateur présente alors un rayon de courbure **R** compris entre 30 mm et 60 mm et, de préférence, pour la partie **15** du guide perforateur **10** s'étendant entre la poignée **14** et l'extrémité **12**, compris entre 40 mm et 50 mm, la partie extrême **16** du guide perforateur **10** présentant alors un rayon de courbure variable.

Selon une autre forme de réalisation du guide perforateur **10,** illustrée aux **fig. 7** à **9****,** le corps allongé **11** du guide **10** présente une extrémité **17** de forme hélicoïdale, également adaptée pour la mise en place des bretelles de suspension dans les zones supérieure ou inférieure des trous obturés. De manière préférée, l'extrémité distale **17** du guide perforateur présente alors la forme d'une portion de spire hélicoïdale s'étendant sur un angle γ compris entre 180° et 360° et, de préférence, compris entre 255° et 270°. De même, de manière préférée, la spire **17** du guide perforateur présente un rayon de courbure compris entre 20 mm et 40 mm, avec un pas compris entre 15mm et 25 mm.

Il doit être noté que selon ces exemples de réalisation les guides perforateurs **10** présentent au niveau de leurs extrémités **12** un chas **19** permettant la fixation des aiguilles **5** pour assurer une traction de l'introducteur **1** dans les tissus où doit être mise en place la bandelette **4**.

Cependant la présence d'un tel chas **19** n'est pas strictement nécessaire à la réalisation d'un guide perforateur selon l'invention.

Ainsi, afin de tenter de réduire au maximum le traumatisme par abrasion des zones tissulaires traversées, il peut être envisagé de mettre en oeuvre un ancillaire ou dispositif de mise en place associant le guide perforateur **10** à une chemise souple **50** de forme complémentaire à celle du guide **10** comme cela est illustré aux **fig. 10** et **11** à **13**. La chemise **50** est engagée sur le guide perforateur **10** qui présente alors une butée ou garde **51** sur laquelle la chemise **50** vient en appui lors de l'introduction du guide perforateur **10** dans le corps de la patiente. La chemise **50** est laissée en place dans le corps de la patiente après retrait du guide perforateur **10** avant la mise place de la bandelette **4** et passe de l'introducteur **10**. La chemise utilisée permet ainsi de créer un canal pour le passage d'un élément de traction **5** de l'introducteur **10** et dans lequel l'aiguille **5** peut être déplacée par glissement, de manière à régler la position de la bandelette **4** sans abrasion des tissus traversés. Il est alors utilisé une chemise **50** pour la mise en place des deux extrémités de la bandelette **4**. Les chemises **50** sont ensuite retirées en même temps que les parties correspondantes de l'introducteur **10.**

Ainsi, la mise en oeuvre des chemises **50** évite les phénomènes inflammatoires aigus et réduit le traumatisme tissulaire, dans la mesure où les sites d'implantation sont composés de tissus musculaires très spécialisés qui ont perdu une grande partie de leurs capacités de régénération et cicatrisation rapide.

Selon les exemples illustrés aux **fig. 10** à **13****,** la chemise souple **50** présente une longueur sensiblement égale à la longueur utile du guide perforateur, à savoir la partie de ce dernier comprise entre sa pointe et la butée ou garde **51**.

Toutefois, dans certains cas et notamment pour des patientes présentant une corpulence importante, lors du retrait du guide perforateur **10**, l'extrémité de la chemise tubulaire **50** peut se trouver à l'affleurement de l'incision d'introduction du guide perforateur, voire en deçà de cette incision, de sorte que le chirurgien ne pourra pas introduire, dans la chemise la bandelette ou son introducteur.

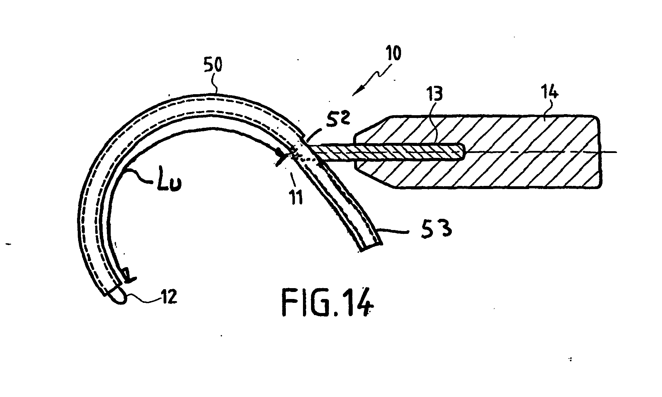

Afin de remédier à cet inconvénient, selon une variante de réalisation de l'invention, la chemise tubulaire **50** présente une longueur supérieure à la longueur **Lu** de la partie utile du guide perforateur **10,** comme cela ressort de la **fig. 14****.** Dans ce cas, la chemise tubulaire **50** présente alors une ouverture latérale **52** qui est aménagée à une distance de l'extrémité libre de la chemise tubulaire, inférieure ou égale et, selon l'exemple illustré, sensiblement égale à la longueur utile **Lu** du guide perforateur. L'ouverture latérale **52** permet alors une mise en place du guide perforateur **10** dans la chemise **50** en laissant la pointe **12** dépasser au niveau l'extrémité de ladite chemise tubulaire.

Ainsi, après mise en place de la chemise tubulaire dans le corps de la patiente, au moyen du guide perforateur, la partie libre **53** de la chemise tubulaire **50** dépasse très largement en dehors du corps de la patiente, de sorte qu'une fois le guide perforateur **10** retiré le chirurgien peut facilement introduire l'introducteur ou un implant dans la chemise tubulaire **50**.

## Revendications

1. - Dispositif pour la mise en place d'une bandelette de renfort (**4**) dans un tissu du corps humain comprenant un introducteur (**1**) qui présente :
■ un corps souple allongé (**2**),
**■** à chacune des deux extrémités du corps, des moyens de traction (**5**),
**■** entre les deux extrémités, une cavité (**3**) de réception de la bandelette (**4**),
**■** et, au niveau de la cavité, des moyens de découpe (**7**) du corps (**2**) en deux parties séparables par une traction exercée sur les moyens de traction (**5**),
**caractérisé en ce que** les moyens de découpe (**7**) comprennent au moins une ouverture (**8**, **8**₁) aménagée dans la paroi de la cavité (**3**) et destinée au passage d'un outil de coupe.

2. - Dispositif selon la revendication 1, **caractérisé en ce que** l'ouverture (**8**), constitutive des moyens de découpe (**7**), s'étend transversalement à l'axe longitudinal (Δ) de la cavité (**3**) et intéresse plus de la moitié de la circonférence de la paroi de la cavité (**3**).

3. - Dispositif selon la revendication 1 ou 2, **caractérisé en ce que** l'ouverture (**8**), constitutive des moyens de découpe (**7**), est adaptée pour autoriser la mise en place de la bandelette (**4**) dans la cavité.

4. - Dispositif selon l'une des revendications 1 à 3, **caractérisé en ce que** les moyens de découpe (**7**) comprennent au moins deux ouvertures (**8**₁) disposées en regard l'une de l'autre.

5. - Dispositif selon l'une des revendications 1 à 4, **caractérisé en ce que** la paroi de la cavité présente une série de perforations (**P**) de stérilisation.

6. - Dispositif selon l'une des revendications 1 à 5, **caractérisé en ce que** les moyens de traction (**5**) comprennent des aiguilles semi-rigides solidaires des extrémités du corps allongé (**2**).

7. - Dispositif selon l'une des revendications 1 à 6, **caractérisé en ce qu**'il comprend une bandelette (**4**) disposée à l'intérieur de la cavité en étant libre.

8. - Dispositif selon des revendications 1 à 7 **caractérisé en ce qu**'il comprend en outre un guide perforateur (**10**) allongé ou trocart dont une extrémité est destinée à être introduite dans le corps d'une patiente et dont l'autre extrémité (**13**) est pourvue d'une poignée (**14**).

9. - Dispositif selon la revendication 8 **caractérisé en ce que** le guide perforateur (10) présente une forme arquée dans un plan.

10. - Dispositif selon la revendication 9 **caractérisé en ce que** la partie arquée (**15**) du perforateur s'étend sur un secteur angulaire supérieur à 140° et, de préférence, inférieur à 180° et, de manière particulièrement préférée, compris entre 150° et 170°.

11. - Dispositif selon la revendication 9 ou 10 **caractérisé en ce que** la partie arquée (**15**) du guide perforateur (**10**) présente alors un rayon de courbure (**R**) compris entre 30 mm et 60 mm et, de préférence, pour la partie du guide perforateur s'étendant entre la poignée et l'extrémité destinée à être introduite dans le corps du patient, compris entre 40 mm et 50 mm.

12. - Dispositif selon la revendication 8 **caractérisé en ce que** le guide perforateur (10) présente, au niveau de son extrémité (**17**) opposée à la poignée (**14**) ou distale, une forme hélicoïdale.

13. - Dispositif selon la revendication 12 **caractérisé en ce que** le guide perforateur (10) présente la forme d'une portion de spire hélicoïdale (**17**) s'étendant sur un angle compris entre 180° et 360° et, de préférence, compris entre 255° et 270°.

14. - Dispositif selon la revendication 13 **caractérisé en ce que** la spire (**17**) du guide perforateur présente un rayon de courbure compris entre 20 mm et 40 mm, avec un pas compris entre 15 mm et 25 mm.

15. - Dispositif selon l'une des revendications 8 à 14 **caractérisé en ce qu'**il comprend en outre une chemise tubulaire amovible (**50**) de forme complémentaire à celle du guide perforateur, destinée à être engagée sur le guide perforateur et à rester dans le corps de la patiente après retrait du guide perforateur (**10**) pour définir un tunnel pour le passage des moyens de traction (**5**) de l'introducteur (**1**).

16. - Dispositif selon la revendication 15 **caractérisé en ce que** la chemise tubulaire (50) présente une longueur supérieure à la longueur utile (**Lu**) du guide perforateur et comprend une ouverture latérale (**52**) pour la mise en place du guide perforateur, l'ouverture latérale (**52**) étant située à une distance d'une extrémité libre de la chemise (**50**) inférieure ou égale à la longueur utile (**Lu**) du guide perforateur.

## Claims

1. A device for the placing a reinforcement band (4) in the tissue of the human body, comprising an introducer (1) which has :
an elongated supple body (2),
traction means (5) at each of the two ends of the body,
between the two ends a cavity (3) for receiving the reinforcement band (4),
and, at the cavity level, means (7) for cutting the body (2) into two separable parts by traction exerted on the traction means (5),
**characterised in that** the cutting means (7) comprise at least one opening (8, 8₁) arranged in the wall of the cavity (3) and intended for passage of a cutting tool.

2. The device according to Claim 1, **characterised in that** the opening (8), constituting the cutting means (7), extends transversally to the longitudinal axis (Δ) of the cavity (3) and takes up more than half of the circumference of the wall of the cavity (3).

3. The device according to Claim 1 or 2, **characterised in that** the opening (8) constituting the cutting means (7) is adapted to allow the reinforcement band (4) to be placed in the cavity.

4. The device according to any of Claims 1 to 3, **characterised in that** the cutting means (7) comprise at least two openings (8₁) placed opposite one another.

5. The device according to any of Claims 1 to 4, **characterised in that** the wall of the cavity has a series of sterilisation perforations (P).

6. The device according to any of Claims 1 to 5, **characterised in that** the traction means (5) comprise semirigid needles solid with the ends of the elongated body (2).

7. The device according to any of Claims 1 to 6, **characterised in that** it comprises a reinforcement band (4) placed freely inside the cavity.

8. The device according to Claims 1 to 7, **characterised in that** it further comprises an elongated or trocar perforator guide (10) whereof one end is intended to be introduced into the body of a patient and whereof the other end (13) is provided with a grip (14).

9. The device according to Claim 8, **characterised in that** the perforator guide (10) has an arched shape in one plane.

10. The device according to Claim 9, **characterised in that** the arched part (15) of the perforator extends over an angular sector greater than 140° and, preferably, less than 180° and, particularly preferably, between 150° and 170°.

11. The device according to Claim 9 or 10, **characterised in that** the arched part (15) of the perforator guide (10) has a radius of curvature (R) of between 30 mm and 60 mm and, preferably, for the part of the perforator guide extending between the grip and the end to be introduced into the body of the patient, of between 40 mm and 50 mm.

12. The device according to Claim 8, **characterised in that** the perforator guide (10) has a helicoidal shape at its end (17) opposite the grip (14) or its distal end.

13. The device according to Claim 12, **characterised in that** the perforator guide (10) has the shape of a helicoidal spire portion (17) extending over an angle between 180° and 360° and preferably between 255° and 270°.

14. The device according to Claim 13, **characterised in that** the spire (17) of the perforator guide has a radius of curvature of between 20 mm and 40 mm, with a pitch of between 15 mm and 25 mm.

15. The device according to any of Claims 8 to 14, **characterised in that** it further comprises a removable tubular sleeve (50) of a shape complementary to that of the perforator guide, to be engaged on the perforator guide and remain in the body of the patient after the perforator guide (10) is removed to define a tunnel for passage of the traction means (5) of the introducer (1).

16. The device according to Claim 15, **characterised in that** the tubular sleeve (50) has a length greater than the useful length (Lu) of the perforator guide and comprises a lateral opening (52) for placing the perforator guide, the lateral opening (52) being located at a distance from a free end of the sleeve (50) less than or equal to the useful length (Lu) of the perforator guide.

## Patentansprüche

1. Vorrichtung für das Einsetzen eines Verstärkungsbändchens (4) in ein Gewebe des menschlichen Körpers, mit einer Einführungsvorrichtung (1), die folgendes aufweist:
- einen langgestreckten flexiblen Körper (2),
- an jedem der zwei Enden des Körpers Zugmittel (5),
- zwischen den zwei Enden einen Hohlraum (3) zur Aufnahme des Bändchens(4),
- und im Bereich des Hohlraums Mittel (7) zum Teilen des Körpers (2) in zwei trennbare Teile durch einen auf die Zugmittel (5) ausgeübten Zug,
**dadurch gekennzeichnet, daß** die Teilungsmittel (7) wenigstens eine Öffnung (8, 8₁) aufweisen, die in der Wand des Hohlraums (3) ausgebildet und für den Durchgang eines Schneidwerkzeugs bestimmt ist.

2. Vorrichtung nach Anspruch 1, **dadurch gekennzeichnet, daß** die einen Bestandteil der Teilungsmittel (7) bildende Öffnung (8) sich quer zur Längsachse (Δ) des Hohlraums (3) erstreckt und mehr als die Hälfte des Umfangs der Wand des Hohlraums (3) betrifft.

3. Vorrichtung nach Anspruch 1 oder 2, **dadurch gekennzeichnet, daß** die einen Bestandteil der Teilungsmittel (7) bildende Öffnung (8) angepaßt ist, um das Einsetzen des Bändchens (4) in den Hohlraum zu gestatten.

4. Vorrichtung nach einem der Ansprüche 1 bis 3, **dadurch gekennzeichnet, daß** die Teilungsmittel (7) wenigstens zwei Öffnungen (8₁) umfassen, die einander gegenüberliegend angeordnet sind.

5. Vorrichtung nach einem der Ansprüche 1 bis 4, **dadurch gekennzeichnet, daß** die Wand des Hohlraums eine Reihe von Sterilisationsperforierungen (P) aufweist.

6. Vorrichtung nach einem der Ansprüche 1 bis 5, **dadurch gekennzeichnet, daß** die Zugmittel (5) halbstarre Nadeln umfassen, die mit den Enden des langgestreckten Körpers (2) fest verbunden sind.

7. Vorrichtung nach einem der Ansprüche 1 bis 6, **dadurch gekennzeichnet, daß** sie ein Bändchen (4) umfaßt, das in dem Hohlraum angeordnet und dabei frei ist.

8. Vorrichtung nach Ansprüchen 1 bis 7, **dadurch gekennzeichnet, daß** sie ferner eine langgestreckte Perforationsführung (10) oder einen Trokar umfaßt, deren bzw. dessen eines Ende dazu bestimmt ist, in den Körper eines Patienten eingeführt zu werden und deren bzw. dessen anderes Ende (13) mit einem Griff (14) versehen ist.

9. Vorrichtung nach Anspruch 8, **dadurch gekennzeichnet, daß** die Perforationsführung (10) eine in einer Ebene gebogene Form aufweist.

10. Vorrichtung nach Anspruch 9, **dadurch gekennzeichnet, daß** sich der gebogene Teil (15) der Perforationsvorrichtung über einen Winkelsektor erstreckt, der größer als 140° und vorzugsweise kleiner als 180° ist und in besonders bevorzugter Weise zwischen 150° und 170° beträgt.

11. Vorrichtung nach Anspruch 9 oder 10, **dadurch gekennzeichnet, daß** der gebogene Teil (15) der Perforationsführung (10) dann einen Krümmungsradius (R) zwischen 30 mm und 60 mm und vorzugsweise - was den Teil der Perforationsführung betrifft, der sich zwischen dem Griff und dem Ende, das in den Körper des Patienten eingeführt werden soll, erstreckt - zwischen 40 mm und 50 mm aufweist.

12. Vorrichtung nach Anspruch 8, **dadurch gekennzeichnet, daß** die Perforationsführung (10) im Bereich ihres dem Griff (14) gegenüberliegenden oder distalen Endes (17) eine Schraubenform aufweist.

13. Vorrichtung nach Anspruch 12, **dadurch gekennzeichnet, daß** die Perforationsführung (10) die Form eines Schraubenwindungsabschnitts (17) aufweist, der sich über einen Winkel von 180° bis 360° und vorzugsweise von 255° bis 270° erstreckt.

14. Vorrichtung nach Anspruch 13, **dadurch gekennzeichnet, daß** die Windung (17) der Perforationsführung einen Krümmungsradius zwischen 20 mm und 40 mm, mit einer Steigung zwischen 15 mm und 25 mm aufweist.

15. Vorrichtung nach einem der Ansprüche 8 bis 14, **dadurch gekennzeichnet, daß** sie ferner einen lösbaren röhrenförmigen Mantel (50) mit einer zu der Form der Perforationsführung ergänzenden Form umfaßt, der dazu bestimmt ist, auf die Perforationsführung aufgesteckt zu werden und nach Herausziehen der Perforationsführung (10) in dem Körper des Patienten zu verbleiben, um einen Tunnel für den Durchgang der Zugmittel (5) der Einführungsvorrichtung (1) zu definieren.

16. Vorrichtung nach Anspruch 15, **dadurch gekennzeichnet, daß** der röhrenförmige Mantel (50) eine Länge aufweist, die größer als die Nutzlänge (Lu) der Perforationsführung ist, und eine Seitenöffnung (52) für das Einsetzen der Perforationsführung aufweist, wobei sich die Seitenöffnung (52) in einem Abstand von einem freien Ende des Mantels (50) befindet, der kleiner als die oder gleich der Nutzlänge (Lu) der Perforationsführung ist.
